# EUROPEAN PATENT SPECIFICATION

(11) **EP 1 267 867 B1**
(45) Date of publication and mention of the grant of the patent: **14.05.2008**
(21) Application number: 00912807.5
(22) Date of filing: 24.03.2000
(51) Int. Cl.: A61K 31/421, A61K 31/5375, A61P 25/06, A61K 31/42, G01N 33/68

(54) **USE OF PROSTANOID EP4 RECEPTOR ANTAGONISTS FOR THE TREATMENT OF HEADACHE AND ASSAYS FOR SUCH ANTAGONISTS**
VERWENDUNG VON PROSTANOID-EP4-REZEPTOR-ANTAGONISTEN ZUR BEHANDLUNG VON KOPFSCHMERZEN UND NACHWEISVERFAHREN FÜR SOLCHE ANTAGONISTEN
UTILISATION DES ANTAGONISTES DU RECEPTEUR EP4 PROSTANOIDE POUR TRAITER LES MAUX DE TETE ET DOSAGES POUR CES ANTAGONISTES

(43) Date of publication of application: 02.01.2003
(73) Proprietor: ASTERAND UK LIMITED, Royston Hertfordshire SG8 5HD (GB)
(72) Inventor: BAXTER, Gordon, Smith, Royston, Hertfordshire SG8 5HD (GB); COLEMAN, Robert, Alexander, Royston, Hertfordshire SG8 5HD (GB); TILFORD, Nicholas, Royston, Hertfordshire SG8 5HD (GB)
(74) Representative: Brasnett, Adrian Hugh
(86) International application number: PCT/GB2000/001138
(87) International publication number: WO 2001/072302

(56) References cited:
- WO-A-00/18405
- WO-A-01/10426
- WO-A-98/55468
- PARSONS, A.A. ET AL: " Effects of prostanoids on human and rabbit basilar arteries precontracted in vitro." CEPHALAGIA, vol. 9, no. 3, 1989, pages 165-171, XP002086056
- COLEMAN, R.A. ET AL: "A novel inhibitory prostanoid receptor in piglet saphenous vein" PROSTAGLANDINS, vol. 47, 1994, pages 151-168, XP000971160 cited in the application
- COLEMAN, R.A. ET AL: "EP4-receptors and cyclic AMP in pig venous smooth muscle: Evidence with agonists and the EP4-antagonist AH22921" ADVANCES IN PROSTAGLANDIN, THROMBOXANE AND LEUKOTRIENE RESEARCH, vol. 23, 1995, pages 241-246, XP000884753 cited in the application
- VAPAATALO, HEIKKI: "Tolfenamic acid and migraine-aspects on prostaglandins and leukotrienes" PHARMACOLOGY AND TOXICOLOGY, vol. 75, no. suppl.2, 1994, pages 76-80, XP000974399
- KOHL, F.V.: "Kopfschmerz durch Arzneimittel" MEDIZINISCHE MONATSCHRIFT FÜR PHARMAZEUTEN, vol. 8, no. 1, 1985, page 2-7 XP000974353
- R. A. COLEMAN, S. P. GRIX, S. A. HEAD, J. B. LOUTTIT, A. MALLETT AND R. L. G. SHELDRICK: "A novel inhibitory prostanoid receptor in piglet saphenous vein" PROSTAGLANDINS, vol. 47, February 1994 (1994-02), pages 151-168,

## Description

### Field of the invention.

The present invention relates to an assay method for agents for the treatment of primary headache disorders and drug-induced headaches in humans and other mammals.

### Background to the invention.

There is a widely held view that the pain of migraine headache originates from abnormally distended blood vessels in the cerebral vasculature. Dilatation in cerebral blood vessels, would cause local pressure resulting in the activation of local sensory pathways and pain. This can be the case also for the other aforementioned primary headache disorders and certain drug-induced headaches.

Many drugs are used to treat primary headache disorders such as migraine, including NSAIDs, ergot alkaloids, and several compounds that interact with different subtypes of 5-hydroxytryptamine (5-HT) receptors either as agonists (e.g. sumatriptan) or antagonists (e.g. ketanserin). However, of the drugs that interact with 5-HT receptors only the class of compounds described as 5-HT_{1D} receptor agonists, of which sumatriptan is an example, will relieve an established headache. 5-HT_{1D} receptor agonists are well known to cause vasoconstriction in the cerebral vasculature which supports the vasodilatation theory [Humphrey, P.P.A., Feniuk, W., Motevalian, M., Parsons A.A. and Whalley, E.T., 'The vasoconstrictor action of sumatriptan on human dura mater in 'Serotonin: Molecular Biology, Receptors and Functional Effects' ed. Fozard, J. and Saxena, P.R., Birkhauser Verlag, Basel, 1991].

Exogenous administration of the potent vasodilator E-series, but not I-series, prostanoids to migraineurs is known to induce migraine-like symptoms [Carlson, L.A., Ekelund, L.G. and Oro, L. (1986) Acta Med. Scand. 183, 423; Peatfield, R. (1981) Headache 32, 98-100]. In menstrual migraines plasma concentrations of prostaglandin E2 (PGE2) are significantly increased during the pain phase of the migraine attack (Nattero, G, et al, 1989, Headache 29; 232-237). Similarly, increased levels of PGE₂ have been found in saliva of common migraine patients during migraine attacks (Obach Tuca, J, et al, 1989, Headache, 29; 498-501).

This evidence, together with the effectiveness of NSAIDS (which act by inhibiting the biosynthesis of prostanoids) in both preventing or relieving a migraine attack [Karachalios, G.N., Fotiadou, A., Chrisikos, N., Karabetsos, A. and Kehagoiglou (1992) Headache 21, 190; Hansen, P. (1994) Pharmacol. Toxicol. 75, Suppl.2, 81-82] supports the involvement of prostanoids in the aetiology of the disease. The precise role of prostanoids is unclear but could involve a combination of local vasodilator, inflammatory, and/or hyperalgesic actions. The prostanoid most often associated with such actions is PGE₂.

Thromboxane A₂ (TXA₂), an active metabolite of arachidonic acid in human platelets, is a potent constrictor of vascular smooth muscle and aggregator of platelets. The compounds AH22191 and AH23848 (see below) and related compounds antagonise the actions of TXA₂ and therefore inhibit platelet aggregation and bronchoconstriction. Hence these compounds have been claimed for use in the treatment of asthma and as anti-thrombotic agents in cardiovascular disorders. GB Patent 2,028,805 and US Patent 4,342,756 describe AH22921 and AH23848, respectively. These compounds have the following structures:

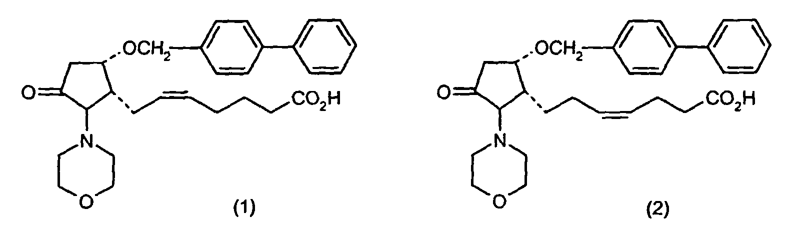

Additionally, both AH22921 and AH23848 have also been shown to be weak antagonists of PGE₂- induced relaxation of piglet saphenous vein (pA₂ values 5.3 and 5.4, respectively) through blockade of EP₄ receptors [Coleman, R.A., Grix, S.P., Head, S.A., Louttit, J.B., Mallett, A. and Sheldrick, R.L.G. (1994) Prostaglandins 47, 151-168; Coleman, R.A., Mallett, A. and Sheldrick, R.L.G. (1995) Advances in Prostaglandin, Thromboxane and Leukotriene Research, 23, 241-246] but have no effect on other EP receptor subtypes EP₁, EP₂ and EP₃.

A large number of PGE₂ antagonists are known. These include oxazole derivatives, such as those disclosed in WO98/55468, dibenzoxazepine derivatives such as those of EP-A-0512399, EP-A-0512400, EP-A-0539977, WO93/09104, WO93/13082, WO94/25456 and WO95/12600, 1,2-diarylcyclopentenyl compounds such as those of US-A-5,344,991, and carboxylic acids and acylsulphonamides such as those of WO99/47497.

### Disclosure of the invention.

We have examined the action of a number of prostanoids on human isolated cerebral blood vessels and made the unexpected discovery that PGE₂ has a complex action on these vessels whereas the other vasodilator prostanoids, PGD₂ and PGF₂ₐ, produce no effects. PGE₂ can cause constriction of larger vessels (>than 1mm diameter), but more significantly we believe, in the context of pain associated with migraine, it has surprisingly been found that it causes a potent concentration-related relaxation of smaller cerebral vessels (<lmm diameter). By studying a variety of pharmacologically active agents this relaxant effect was found to be mediated by prostanoid EP₄ receptors. Further experiments carried out in human coronary and pulmonary arteries have shown that PGE₂ lacks this dilatory effect in these tissues. Therefore, and in contrast with current anti-migraine drug treatments, it is not expected that EP₄ receptor antagonists will cause significant cardiovascular problems.

Thus our findings described herein are consistent with the novel theory that relaxation of small cerebral arteries by PGE₂ is mediated via EP₄ receptors. Thus EP₄ receptor antagonists, particularly selective EP₄ antagonists, are useful in preventing the relaxation of such arteries.

We therefore believe this unexpected action of PGE₂ could account for the pain in migraine. Preventing increased blood flow to these small cerebral arteries has positive implications in the treatment of migraine and drug induced headaches. Thus an EP₄ receptor antagonist, particularly a selective EP₄ receptor antagonist, may provide a novel and effective anti-migraine agent with advantages over existing therapies, especially NSAIDS. As well as less side effect liability, an EP₄ receptor antagonist should exhibit greater efficacy than an NSAID because an NSAID would eliminate both the detrimental vasodilator and beneficial vasoconstrictor effects on cerebral vasculature caused by endogenous prostaglandins. In contrast, an EP₄ receptor antagonist should only inhibit the detrimental vasodilator effect.

The surprising finding that EP₄ receptor-mediated dilatation of cereberal blood vessels is a major pathway in the induction of primary headache disorders provides novel assay methods for the identification and validation of therapeutic agents.

Thus the invention provides an assay for an agent which is an EP4 receptor antagonist for the treatment of a primary headache disorder or drug-induced headache, which assay comprises:
(a) providing a human EP4 receptor;
(b) bringing a potential agent for said treatment into contact with said receptor;
(c) determining whether said agent has a binding affinity for the human EP4 receptor at least 10-fold higher than for the EP1, EP2 and EP3 receptors; and
(d) testing the agent so selected for safety and/or toxicity in a non-human animal subject.

In the above-mentioned assay, the EP4 receptor may be either a recombinant human EP4 receptor or an EP4 receptor on isolated vasculature from post-mortem human sources.

### Description of the Drawings.

Figure 1 shows concentration-related relaxation of pre-contracted cerebral blood vessels by PGE₂.
Figure 2 shows concentration-related relaxation by PGE₂ of cerebral blood vessels pre-contracted by (A) U46619, and (B) and (C), 5-HT.
Figure 3 shows the effect of prostanoids PGD₂ and PGP₂₀ on smaller diameter cerebral blood vessels.
Figure 4 shows the relaxant response of cerebral blood vessels to iloprost and cicaprost.
Figure 5 shows the effect of EP2 receptor antagonists on the relaxant response of cerebral blood vessels.
Figure 6 shows the role of EP₄ receptors in PGE₂-mediated relaxation of cerebral arteries in the presence of a receptor antagonist.
Figure 7 shows the effect of PGE₂ on pre-contracted preparations of pulmonary (Fig. 7A) or coronary (Fig. 7B) artery.

### Detailed description of the invention.

### Disorders.

As used herein, the term "primary headache disorder" includes migraine, tension-type headache, cluster headache, analgesic rebound headache, chronic paroxysmal hemicrania and headache associated with vascular disorders.

In a preferred aspect, the invention relates to an assay for agents for treating migraine. Migraine attacks are classified as migraine with- or migraine without aura. Although diagnostic criteria are somewhat different the (drug) treatment is the same. Migraine without aura is described as: idiopathic, recurring headache disorder, manifesting in attacks lasting 4-72 hours, in which headaches are typically unilateral, throbbing, of moderate to severe intensity, aggravated by routine physical activity, and accompanied by nausea and intolerance to brightness and noise. Migraine with aura is described as: idiopathic, recurring disorder manifesting with attacks of neurological symptoms unequivocally localisable to cerebral cortex or brain stem, usually developing over 5-20 minutes and lasting less than 60 minutes, and followed or accompanied by migraine headache and its associated features.

Drug-induced headache, particularly ergotamine-induced headache, is a common problem in migraine treatment. Some case reports suggest that even the new serotonergic antimigraine drugs such as sumatriptan can lead to overuse and subsequent drug-induced headache.

### "EP₄ receptor antagonist".

For the avoidance of doubt, in the context of this invention, an EP₄ receptor antagonist is any compound, agent or mixture showing antagonist activity at EP₄ receptors, including and especially antagonist activity against PGE₂ induced relaxation of human isolated cerebral blood vessels.

In any of the above aspects of the invention the EP₄ receptor antagonist is a chemical entity that blocks the activity of PGE₂ at the (human) EP, receptor or better, any chemical entity that competes with PGE₂, or any other EP₄ receptor ligand, for the EP₄ receptor binding site (preferably in a competitive manner) and does not exert any activity itself at the EP₄ receptor.

A medicament suitable for use in the treatment of primary headache disorders or drug induced headaches, may be an oxazole compound of formula (1): 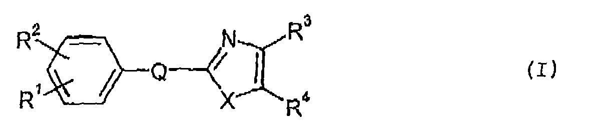 wherein R¹ is lower alkyl substituted with hydroxy, protected carboxy or carboxy; carboxy; protected carboxy; carbamyol; a heterocyclic group; cyano; hydroxy; halo(lower)alkylsulfonyloxy; lower alkoxy optionally substituted with hydroxy or carbamoyl; aryl substituted with carboxy, protected carboxy, carbamoyl or a heterocyclic group; or amino optionally substituted with protected carboxy or lower alkylsulfonyl,
R² is hydrogen or lower alkyl,
R³ is aryl optionally substituted with halogen,
R⁴ is aryl optionally substituted with halogen,
Q is 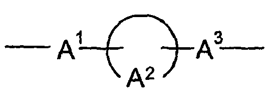 in which -A¹- is a single bond or lower alkylene, 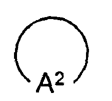 is cyclo (C₅-C₉) alkane, bicyclo (C₆-C₉) alkane or bicycle (C₅-C₉) alkane and -A³- is a single bond or lower alkylene, and
X is O, NH or S;
or a salt or its solvate thereof.

The compounds of formula (I) may contain one or more asymmetric centres and thus they can exist as enantiomers or diastereoisomers. Furthermore certain compounds of formula (I) which contain alkenyl groups may exist as cis- or trans-isomers. In each instance, mixtures and separate individual isomers may be prepared.

The compounds of the formula (I) may also exist in tautomeric forms and the invention includes both mixtures and separate individual tautomers.

The compound of the formula (I) and its salt can be in a form of a solvate, which is included within the scope of the present invention. The solvate preferably include a hydrate and an ethanolate.

The term "lower" is intended to mean 1 to 6 carbon atom(s), unless otherwise indicated.

Suitable "lower alkyl" and lower alkyl moiety in the term "halo(lower)alkylsulfonyl" and "lower alkysulfonyl" may include straight or branched one having 1 to 6 carbon atom(s), such as methyl, ethyl, propyl, isopropyl, butyl, isobutyl, sec-butyl, t-butyl, pentyl, t-pentyl, hexyl or the like, preferably one having 1 to 4 carbon atom(s).

Suitable "lower alkylene" may include straight or branched one having 1 to 6 carbon atom(s), such as methylene, ethylene, trimethylene, tetramethylene, pentamethylene and hexamethylene, preferably one having 1 to 3 carbon atoms(s), more preferably methylene.

Suitable "cyclo(C₃-C₉)alkane" may include cyclopropane, cyclopentane, cyclohexane, cycloheptane, cyclooctane, or the like, preferably one having 5 to 7 carbon atoms.

Suitable "cyclo(C₅-C₉)alkene" may include cyclopentene, cyclohexene, cycloheptene, cyclooctene, or the like, preferably one having 5 to 7 carbon atoms.

Suitable "bicyclo(C₅-C₉)alkane" may include bicycloheptane (e.g., bicyclo[2.2.1]heptane, etc.), bicyclooctene (e.g., bicyclo[3.2.1]octane, etc.), or the like.

Suitable "bicyclo(C₆-C₉)alkene" may include bicycloheptene (e.g., bicyclo[2.2.1]hept-2-ene, etc.), bicyclooctene (e.g., bicyclo[3.2.1]oct-2-ene, etc.), or the like.

Suitable "aryl" may include phenyl, lower alkylphenyl' (e.g., tolyl, ethylphenyl, propylphenyl, etc.), naphthyl or the like.

Suitable "heterocyclic group" may include one containing at least one hetero atom selected from nitrogen, sulfur and oxygen atom, and may include saturated or unsaturated, monocyclic or polycyclic group, and preferably one may be heterocyclic group such as 3 to 6-membered heteromonocyclic group containing 1 to 4 nitrogen atoms, for example, pyrrolyl, pyrolinyl, imidazolyl, pyrazolyl, pyridyl, pyrimidinyl, pyrazinyl, pyridazinyl, triazolyl (e.g., 4H-1,2,4-triazolyl, 1H-1,2,3-triazolyl, 2H-1,2,3-triazolyl, etc.), tetrazolyl (e.g., 1H-tetrazolyl, 2H-tetrazolyl, etc.), or the like, more preferably tetrazolyl.

Suitable "lower alkoxy" may include methoxy, ethoxy propoxy, isopropoxy, butoxy, isobutoxy, t-butoxy, pentyloxy, t-pentyloxy, hexyloxy, or the like, preferably methoxy.

Suitable "protected carboxy" may include esterified carboxy or the like.

Suitable example of the ester moeity of an esterified carboxy may be the ones such as lower alkyl (e.g., methyl, ethyl, propyl, isopropyl, butyl, isobutyl, tert-butyl, pentyl, hexyl, etc.) which may have at least one suitable substituent(s), for example, lower alkanoyloxy(lower)alkyl [e.g., acetoxymethyl, butyryloxymethyl, valeryloxymethyl, pivaloyloxymethyl, etc.], halo(lower)alkyl (e.g., 2-iodoethyl, 2,2,2-trichloroethyl, etc.); lower alkenyl (e.g., vinyl, allyl, etc.); lower alkynyl (e.g., ethynyl, propynyl, etc.); ar(lower)alkyl which may have at least one suitable substituent(s) (e.g., benzyl, 4-methoxybenzyl, 4-nitrobenzyl, phenethyl, trityl, etc.); aryl which may have at least one suitable substituent(s) (e.g., phenyl, tolyl, 4-chlorophenyl, tert-butylphenyl, xylyl, mesityl, cumenyl, etc.); phthalidyl; or the like.

Suitable "halo" group in the term of "halo(lower)alkylsulfonyl" may include fluoro, chloro, bromo, iodo, or the like.

Suitable "halo(lower)alkylsulfonyloxy" may include trifluoromethanesulfonyloxy, or the like.

Preferred embodiments of the azole compounds (I) are as follows:
- R¹: is lower alkyl substituted with carboxy; carboxy; protected carboxy; carbamoyl; a heterocyclic group; lower alkoxy substituted with carbamoyl; aryl substituted with carboxy, carbamoyl or a heterocyclic group; or amino optionally substituted with lower alkylsulfonyl (more preferably lower alkyl substituted with carboxy; carboxy; carbamoyl; tetrazolyl; lower alkoxy substituted with carbamoyl, aryl substituted with carboxy or carbamoyl),
- R²: is hydrogen or lower alkyl,
- Q: is 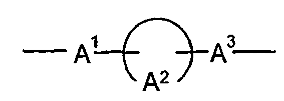 in which -A¹- is a single bond or lower alkylene (more preferably methylene), 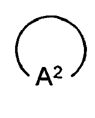
is cyclo(C₅-C₉)alkene, cyclo (C₃-C₉) alkane or bicyclo (C₆-C₉) alkene, bicyclo(C₅-C₉)alkane (more preferably cyclo(C₅-C₇)alkene, cyclo(C₅-C₇)alkane, byciclo[2.2.1]heptane or byciclo[2.2.1]heptane), and -A³- is a single bond or lower alkylene (more preferably single bond) and X is 0.

A compound of the formula (I) is 3-([2-(4,5-diphenyloxazol-2-yl)-2-cyclohexen-1-yl]methyl)benzoate, or a salt thereof, particularly the sodium salt.

Suitable salts of the compound of formula (I) are pharmaceutically acceptable conventional non-toxic salts and include a metal salt such as an alkali metal salt (e.g. a sodium or potassium salt) and an alkaline earth metal salt (e.g. a calcium or magnesium salt), an ammonium salt, an organic base salt (e.g., a trimethylamine salt, triethylamine salt, pyridine salt, picoline salt or a dicyclohexylamine salt), an organic acid salt (e.g., an acetate, maleate, tartrate, methanesulfonate, benzenesulfonate, formate, toluenesulfonate or trifluoroacetate salt), an inorganic acid salt (e.g., a hydrochloride, hydrobromide sulfate or phosphate), or a salt with an amino acid (e.g., arginine, aspartic acid or glutamic acid).

Compounds of the formula (I), and processes for their production are described in WO98/55468. This citation discloses that these compounds, including salts and solvates thereof, are EP₄ receptor antagonists. Although a large number of therapeutic uses of these compounds are described, these do not include the treatment of primary headache disorders, including migraine.

### Selective EP₄ receptor antagonist

The assay of the invention is suitable for identification of a selective EP₄ receptor antagonist. By this it is meant that the antagonist has a binding affinity for the EP₄ receptor which is at least 10-fold higher than for the receptors EP₁, EP₂ and EP₃. With respect to EP₃, we have also found that PGE₂ causes contraction of cerebral arteries via interaction with SP₃ receptors.

The binding of an antagonist to the EP₄ receptor may be determined by competition against PGE₂. For example, the EP₄ receptor may be provided as a recombinantly produced receptor expressed in human cell lines. The murine and human EP₄ receptors have been cloned (Honda et al J. Biol. Chem., 1993, 268; 7759-7762; and An et al, Biochem. Biophys. Res. Commun., 1993, 197; 263-270), although these were initially characterised in error as EP₂ receptors (see review by Coleman, R. A., Prostanoid Receptors, Classification, Characterisation and Therapeutic Relevance, in Eicosanoids: From Biotechnology to Therapeutic Applications, eds. Folco, Samuelsson, Maclouf & Velo., 1996, Plenum Press, New York, pages 137-154), the text of which is also imported herein by reference and forms an integral part of this disclosure.

A method of identifying and quantifying EP₄ receptor antagonists is described in the two publications by Coleman, R.A., 1994 and 1995, listed above. The entire text of these publications is hereby imported by reference and forms an integral part of this disclosure and the inventive concepts described.

In one method, EP₄ receptor antagonists may be characterised by providing a natural source of the receptors, such as piglet saphenous vein. Sections of vein from freshly killed animals may be cut into rings of 4-5 mm width and suspended in an organ bath in Krebs solution. Changes in vessel tension in response to test compounds may be determined by isometric transducers connected to a suitable recording device. The tissue may be contracted, e.g. with phenyleprine, and the relaxant effect of increasing concentrations of PGE₂ determined. The relaxant effect may be determined in the presence or absence of potential antagonists, with a shift in the concentration of PGE₂ required to provide an specified degree of relaxation being indicative of an antagonistic effect.

In another method, EP₄ receptor antagonists may be characterised using sections of cerebral artery. This is because we have found that this is the predominant PGE₂ relaxant receptor in this blood vessel. Sections of cerebral artery are removed from different regions of preparations of human cerebral vasculature containing an intact circle of Willis. Intact rings of this cerebral artery, 2-3mm in length, are set up under isometric conditions in 10 ml organ baths under an initial tension of lg. All tissues are maintained at 37°C and gassed constantly with 95% O₂ / 5% CO₂. Following a 90 min equilibration period all tissues are challenged with phenylephrine (1 µM), to determine tissue viability. Once a stable contraction is obtained, tissues are exposed to a range of prostanoid receptor agonists, in the absence or presence of receptor antagonists, to determine the functional role of prostanoid receptors in maintaining arterial tone.

### Combined therapies.

EP₄ receptor antagonists selected by an assay of the invention may, if desired, be used in combination with one or more other therapeutic agents. The other therapeutic agent(s) may be an agent active against a primary headache disorder, or an agent whose side-effects may induce a primary headache disorder, such as a chemotherapeutic agent. Agents for the treatment of a primary headache disorder include an ergot derivative, for example dihydroergotamine, a 5-HT₂ receptor antagonist, for example ketanserin, or a 5-HT_{1D} receptor agonist, for example sumatriptan, naratriptan or zolmitriptan, a β-blocker for example propranolol, or a non-steroidal anit-inflammatory drug, such as asprin, paracetamol (acetaminophen) or ibuprofen.

### Assay methods.

The invention provides an assay method for an agent for the treatment of a primary headache disorder or drug-induced headache, which assay is as defined herein above.

The mode of interaction of the agent with the EP₄ receptor will be determined according to the format of the assay, which may be varied within the routine skill and knowledge of those of skill in the art. For example, in one aspect the assay may simply determine the binding of the agent to the EP₄ receptor. There are numerous ways in which such an assay could be performed. For example, the receptor may be provided on a solid support, and the binding of the agent determined in a competitive assay in which the agent, or a competitor (e.g. PGE₂ or a known EP₄ receptor antagonist) is labelled, so that the displacement of the competitor by the agent may be determined as an indication of binding. Other binding formats, for example in which the receptor is labelled, may be provided within the ordinary skill and knowledge of those in the art.

Alternatively, the assay may be one in which the response of EP₄ receptors in a biological system is determined. The receptors may be provided on tissue which naturally expresses these receptors. For example, the receptor may be provided on isolated vasculature, such as cerebral arteries. The vasculature may be isolated from any suitable source, e.g. post-mortem human sources, or animal sources, such as pigs, rats, rabbits and the like. Alternatively, the receptor may be provided by recombinant expression from an EP₄ receptor cDNA in a suitable host cell expression system, The biological response to the agent may be determined, e.g. to see if the agent antagonises the response to PGE₂ or the like.

In a preferred aspect, the EP₄ receptor is provided in step (a) together with at least one other receptor selected from the group of EP_{1,} EP₂, EP₃, TP, IP and DP receptors, Alternatively, the assay is run in parallel or sequential to (either before or after) with an assay to determine the interaction of the agent to one of these other prostanoid receptors. In this aspect, the determining step may include a comparison of the activity or affinity of the agent against the other EP or other prostanoid receptor types so as to determine whether or not the antagonist is a selective EP₄ receptor antagonist.

In a particularly preferred embodiment, the affinity of the putative EP₄ receptor antagonist to the EP₃ receptor is determined in the presence of a preparation of EP₃ receptors. For example, the affinity of binding of an EP₄ receptor antagonist to the EP₃ receptor may be determined using a selective radioligand to the EP₃ receptor. Preferably, the test selected as an EP₄ receptor antagonist will show lower affinity to the EP₃ receptor than to the EP₄ receptor.

Agents selected by the assays of the invention may then be subject to one or more of the following steps:
(e') testing the agent so selected in a human patient for efficacy in treating a primary headache disorder;
(e'') formulating the agent with one or more carriers, diluents or second agents for the treatment of primary headache disorders.

Where other receptors are used in assays according to the invention, these may also be supplied in recombinant form. The cloning of these receptors are described in the following citations, the disclosures of which are incorporated herein by reference.
- DP:: Boie, Y., Sawyer, N., Slipetz, D.M., Metters, K.M. and Abramovitz, M (1995) Molecular Cloning and characterization of the human prostanoid DP receptor. J. Biol. Chem. 270, 18910-18916.
- EP1:: Funk, C.D., Furci, L., FitzGerald, G.A., Grygorczyk, R., Rochette, C., Bayne, M., Abramovitz, M., Adam, M. and Metters, K.M. (1993) Cloning and expression of a cDNA for the human prostaglandin E receptor EP1 subtype. J. Biol. Chem. 268, 26767-26772
- EP2:: Regan, J.W., Bailey, T.J., Pepperl, D.J., Pierce. K.L., Bogardus, A.M., Donello, J.E., Fairbairn, C.E., Kedzie, K.M., Woodward, D.F. and Gil, D.W. (1994) Cloning of a novel human prostaglandin receptor with characteristics of the pharmacologically defined EP2 subtype. Mol. Pharmacol. 40, 213-220.
- EP3:: Regan, J.W., Bailey, T.J., Donello, J.E., Pierce, K.L., Pepperl, D.J., Zhang, D., Kedzie, K.M., Fairbarin, C.E., Bogardus, A.M., Woodward, D.F. and Gil, D.W. (1994) Molecular cloning and expression of human EP3 receptors: presence of three variants with differing carboxyl termini. Br. J. Pharmacol. 112, 377-385.
- TP:: Hirata, M., Hayishi, Y., Ushikubi, F., Yokota, Y., Kageyama, R., Nakanishi, S. and Narumiya, S. (1991) Cloning and expression of the human thromboxane A2 receptor. Nature 349, 617-620.
- IP:: Boie, Y., Rushmore, T.H., Darmon-Goodwin, A., Grygorczyk, R., Slipetz, D.M., Metters, K.M. and Abramovitz, M. (1994) Cloning and expression of a cDNA for the human prostanoid IP receptor. J. Biol. Chem. 269, 12173-12178.

In preferred aspects of this part of the invention, the EP₄ receptor is provided together with, or in parallel with, at least one other EP receptor, preferably at least the EP₃ receptor. The other receptor(s) may be provided in the various forms (e.g. isolated on a solid support, in tissue on which it occurs naturally or recombinantly) mentioned above. Conveniently, the assay is performed on vasculature which contains the EP₄ receptor together with any other desired receptor.

Compounds of the formula (I) as defined above, may be used in assays of the invention in order to select an agent which is a selective EP₄ receptor antagonist. Other agents which may be used in performing assays of the invention include other compounds such as PGE₂ antagonists described in the patent applications cited above. Further, libraries of small molecules are commercially available and such libraries may be used in assays of the invention.

### Formulation and administration of EP₄ receptor antagonists.

The EP₄ receptor antagonists may be administered as the raw chemical but the active ingredients are preferably presented as a pharmaceutical formulation. Suitable pharmaceutical formulations are described in the above referenced patent specifications.

Thus, the EP₄ antagonists may be formulated for oral, buccal, parenteral, topical, depot or rectal administration or in a form suitable for administration by inhalation or insufflation (either through the mouth or nose). Oral and parenteral formulations are preferred.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); filters (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxbenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or lozenges formulated in conventional manner.

The EP₄ antagonists may be formulated for parenteral administration by bolus injection or continuous infusion. Formulations for injection may be presented in unit dosage form, e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The EP₄ antagonists may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The EP₄ antagonists may also be formulated in rectal compositions such as suppositories or retention enemas, e.g. containing conventional suppository bases such as cocoa butter or other glycerides.

The EP₄ antagonists may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the EP₄ antagonists may be formulated as solutions for administration via a suitable metered or unit dose device or alternatively as a powder mix with a suitable carrier for administration using a suitable delivery device.

Suitable dose ranges may be calculated by those skilled in the art in light of toxicological data. It will be appreciated that it may be necessary to make routine variations to the dosage, depending on the age and condition of the patient, and the precise dosage will be ultimately at the discretion of the attendant physician or veterinarian. The dosage will also depend on the route of administration and the particular compound selected. A suitable dose range is, for example, 0.01 to 100 mg/kg, such as from 0.01 to about 50mg/kg bodyweight, 1 to 4 times per day.

The invention is illustrated by the following examples, which shows that small cerebral arteries have EP₄ receptors.

### PGE₂ causes dilatation of middle and anterior cerebral arteries in vitro, via interaction with EP₄ receptors.

### Materials and Methods.

Sections of cerebral artery were removed from different regions of preparations of human cerebral vasculature containing an intact circle of Willis. Intact rings of this cerebral artery, 2-3mm in length, were set up under isometric conditions in 10 ml organ baths under an initial tension of 1g. All tissues were maintained at 37°C and gassed constantly with 95% O₂ / 5% CO₂. Following a 90 min equilibration period all tissues were challenged with phenylephrine (1 µM), to determine tissue viability. Once a stable contraction had been obtained, tissues were exposed to a range of prostanoid receptor agonists, in the absence or presence of receptor antagonists, to determine the functional role of prostanoid receptors in maintaining arterial tone.

### Results

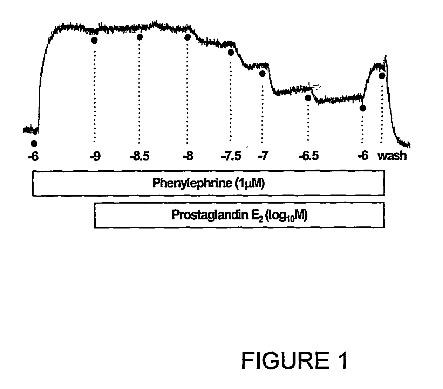

The effects of prostanoid activation were determined in varying sizes of human cerebral artery. On larger vessels (internal diameter > 1mm), PGE₂ generally caused a concentration-related contraction, whereas on smaller vessels (internal diameter < 1mm), it consistently caused potent concentration-related relaxation of pre-contracted cerebral blood vessels. This is shown in Figure 1, which is a trace showing the relaxant effects of PGE₂ on a cerebral blood vessel in an isolated tissue chamber. The isolated vessel has been pre-contracted with phenylephrine and exposed to increasing concentrations of PGE₂. Maximum relaxation occurs below 10⁻⁶M and can be maintained until it is washed out.

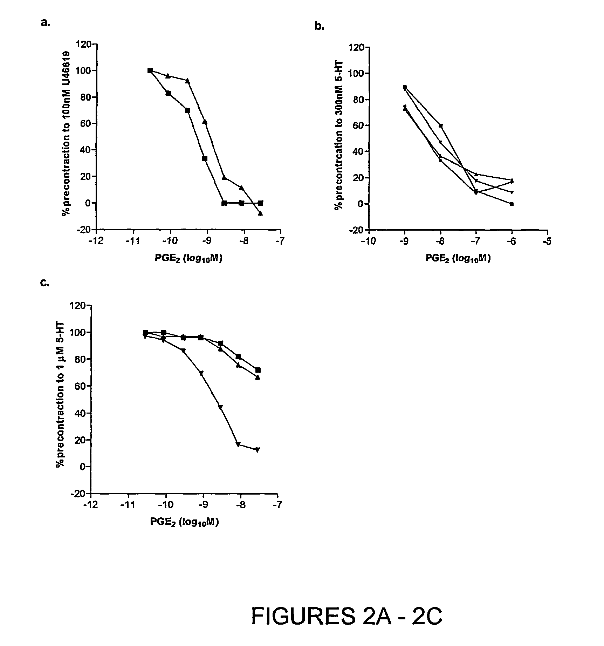

Additionally PGE₂ was shown to induce maximal or near maximal relaxation of cerebral artery rings pre-contracted with the TP receptor agonist U46619 (11α,9α-epoxymethano-PGH₂), (100nM) or 5-hydroxytryptamine (5-HT), (300nM and 1µM). This is shown in Figure 2, in which is shown the cumulative concentration-effect curves to the relaxant effects of PGE₂ on human cerebral artery rings pre-contracted with: (A) U46619 (100nM); (B) 5-HT (300nM) and (C) 5-HT (1 µM). Each curve represents a different tissue.

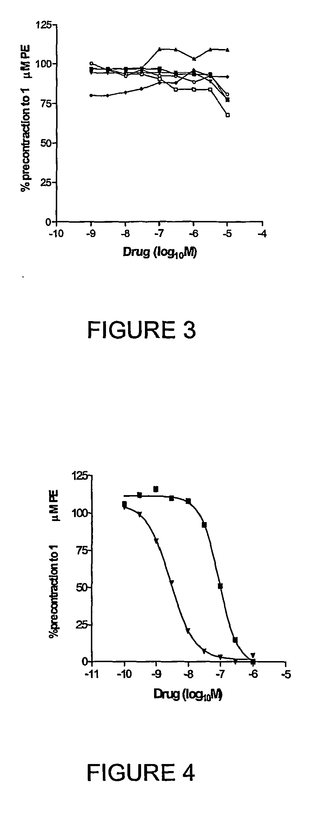

On smaller diameter cerebral blood vessels, the closely related prostanoids, PGD₂ and PGF_{2α}, were found to be without effect, as illustrated in Figure 3, which shows the cumulative concentration-effect curves to PGD₂ (open symbols) and PGF_{2α}(closed symbols) on human cerebral artery rings contracted with phenylephrine (1 µM). GR32191 (4-heptenoic acid, 7-[5-([1,1'-biphenyl]-4-ylmethoxy)-3-hydroxy-2-(1-piperidinyl)-cyclopentyl]-,hydrochloride, [1R[1.alpha. (Z), 2.beta., 3.beta., 5.alpha.]]), (1 µM), was present in bathing solution to block prostanoid TP-receptors. Each curve represents a different tissue.

The presence of IP receptors on human cerebral artery rings was also investigated. Iloprost and cicaprost induced relaxation in tissues previously contracted with phenylephrine. It was therefore essential that any involvement of IP receptors in mediation of the effects of PGE₂ was excluded when assessing antagonist effects at EP receptors. The results of an experiment showing this is set out in Figure 4, which illustrates the cumulative concentration-effect curves to iloprost (closed squares) and cicaprost (closed triangles) on human cerebral artery rings contracted with phenylephrine (1 µM). GR32191 (1 µM) was present in bathing solution to block prostanoid TP-receptors. However, PGE₂ is at least 100-fold less potent at IP receptors compared to iloprost and cicaprost, and therefore relaxation due to IP receptor activation is unlikely to be a major component of PGE₂ relaxant response.

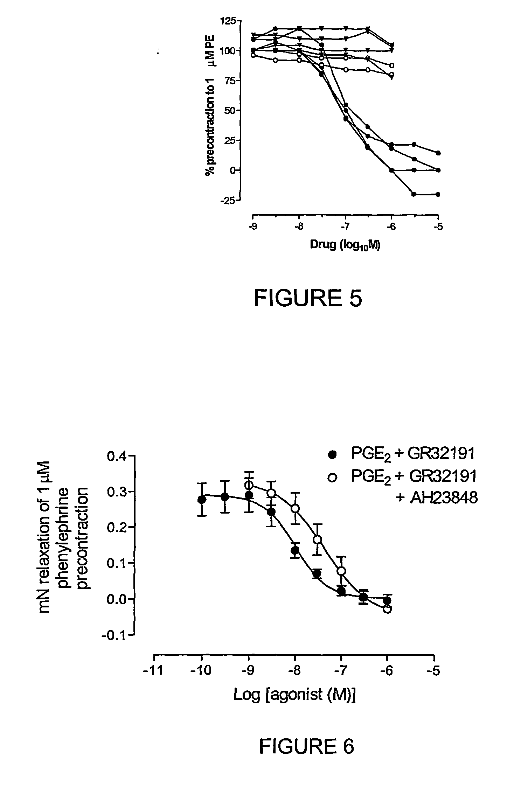

The above results indicate that the high relaxant potency of PGE₂ in small diameter cerebral arteries is indicative of the involvement of an EP receptor. Such inhibitory effects are invariably associated with either the EP₂ or the EP₄ receptor isoforms (Coleman, Smith & Narumiya, 1994, ibid). The EP isoform mediating this effect was determined by receptor exclusion studies using antagonists and agonists selective for other members of the prostanoid receptor family. Butaprost and AH13205 (*trans*-2-(4-[1-hydroxyhexyl]phenyl)-5-oxocyclopentaneheptanoate) are recognised as selective EP₂ agonists and thus their effect on human cerebral artery rings contracted with phenylephrine (1 µM) was determined. The results are shown in Figure 5, which shows the cumulative concentration-effect curves to PGE₂ (closed circles), AH13205 (open circles) and butaprost (closed triangles). GR32191 (1 µM) was present in the bathing solution to block prostanoid TP-receptors.

It can be seen from Figure 5 that neither compound caused relaxation of human cerebral arteries pre-contracted with phenylephrine (Figure 5), excluding EP₂ receptor involvement in the relaxant response to PGE₂. These data provide support that the PGE₂ effect seen in this tissue is mediated via an EP₄ receptor or alternatively, a novel EP receptor(s) yet to be identified. The EP₄ receptor is proposed to be located on the vascular smooth muscle since in a number of experiments removal of the endothelium did not affect the relaxant response to PGE₂.

### Effect of the EP4 receptor antagonist, AH23848.

The role of the EP₄ receptor in PGE2-mediated relaxation of phenylephrine pre-contracted middle cerebral rings was demonstrated using the putative EP4 receptor antagonist AH23848. As a control, cerebral rings were pre-contracted with 1 mM phenylephrine, and concentration-dependently relaxed with PGE2 in the presence of 1mM GR32191. Representative mean ± s.e.m data of 4 middle cerebral artery rings from one donor are shown in Figure 6, closed circles. To test the effect of the EP₄ receptor antagonist AH23848, cerebral rings were preincubated for 45 mins with 10 mM AH23848. AH23848 caused a significant rightward shift (P=0.004, 2 tailed T-Test) in the PGE2-mediated relaxation (Log EC50 (M) PGE2 7.87 ± 0.07; PGE2+ 10 mM AH23848 -7.19 ± 0.09) - Figure 6, open circles.

### Effect of PGE₂ on coronary and pulmonary arteries.

It has also been found that PGE₂ failed to cause relaxation of coronary artery and pulmonary artery which had been precontracted with submaximal concentrations of U46619 or phenylephrine. Rings (2-4mm internal diameter) were prepared from sections of pulmonary or coronary artery (n=3 each).
They were mounted in organ baths under isometric conditions, in gassed Krebs solution (containing indomethacin) at 37°C, and 1-1.5g initial tone.

After at least 60min equilibration, tone was induced with U-46619 (10-100nM) in pulmonary artery or either phenylephrine (1-10µM) or endothelin-1 (-7M) in coronary artery. After a stable plateau had been obtained, tissues received a cumulative concentration effect curve to PGE₂, at log dose intervals, with at least 3 minutes at each concentration. After a maximum response had been obtained, all tissues were treated with cicaprost (0.1-1µM), to induce relaxation.

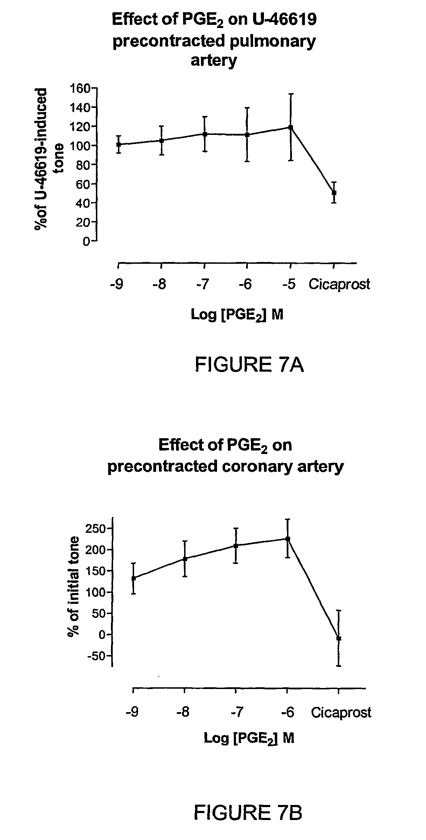

Application of U-46619 (pulmonary artery, see Figure 7A) or phenylephrine or endothelin-1 (coronary artery, see Figure 7B) induced significant increases in basal tone in all preparations. After a stable response had been obtained, application of PGE₂ did not cause relaxation in any preparation, in either coronary or pulmonary artery. The data of Figure 7 are shown as mean ±s.e. mean for n=3 donors.

Thus application of PGE₂ failed to induce relaxation of either pulmonary or coronary artery preparations. A small contraction was induced by PGE₂ in coronary artery, which is likely to be due to the activation of EP₃ receptors. Application of cicaprost induced a relaxation in all preparations, indicating that the tissues were viable and able to exhibit relaxatory responses. No evidence of inhibitory EP receptors could be found.

Thus assays of the invention which are configured to select an EP₄ receptor antagonist which is selective with respect to EP₃ receptors are of particular interest.

## Claims

1. An assay for an agent which is an EP4 receptor antagonist for the treatment of a primary headache disorder or drug-induced headache, which assay comprises:
(a) providing a human EP4 receptor;
(b) bringing a potential agent for said treatment into contact with said receptor;
(c) determining whether said agent has a binding affinity for the human EP4 receptor at least 10-fold higher than for the EP1, EP2 and EP3 receptors; and
(d) testing the agent so selected for safety and/or toxicity in a non-human animal subject.

2. An assay as claimed in claim 1 in which the EP4 receptor is either a recombinant human EP4 receptor or is an EP4 receptor on isolated vasculature from post-mortem human sources.

## Patentansprüche

1. Assay für ein Mittel, welches ein EP4-Rezeptor-Antagonist für die Behandlung einer Primär-Kopfschmerz-Störung oder Arzneistoff-induzierten Kopfschmerzen ist, wobei das Assay folgendes umfasst:
(a) das Bereitstellen eines Human-EP4-Rezeptors;
(b) das In-Kontakt-Bringen eines potentiellen Mittels mit dem Rezeptor für die Behandlung;
(c) das Bestimmen, ob das Mittel eine Bindungsaffinität für den humanen EP4-Rezeptor aufweist, die mindestens 10-fach höher als für die EP1-, EP2- und EP3-Rezeptoren ist; und
(d) das Testen des so ausgewählten Mittels bezüglich der Sicherheit und/oder Toxizität bei einem nicht-humanen tierischen Subjekt.

2. Assay, wie in Anspruch 1 beansprucht, bei dem der EP4-Rezeptor entweder ein rekombinanter EP4-Rezeptor oder ein EP4-Rezeptor auf einem isolierten Gefäßsystem von postmortalen humanen Quellen ist.

## Revendications

1. Analyse pour un agent qui est un antagoniste du récepteur EP4 pour le traitement d'un trouble de céphalée primaire ou d'une céphalée d'origine médicamenteuse, laquelle analyse comprend les étapes consistant à :
(a) fournir un récepteur EP4 humain ;
(b) amener un agent potentiel pour ledit traitement en contact avec ledit récepteur ;
(c) déterminer si ledit agent a une affinité de liaison pour le récepteur EP4 humain au moins 10 fois supérieure à celle pour les récepteurs EP1, EP2 et EP3 ; et
(d) mettre à l'essai l'agent ainsi sélectionné pour la sécurité et/ou la toxicité chez un sujet animal non humain.

2. Analyse selon la revendication 1, dans laquelle le récepteur EP4 est soit un récepteur EP4 humain recombiné, soit un récepteur EP4 d'un système vasculaire isolé provenant de sources humaines post-mortem.
